# EUROPEAN PATENT APPLICATION

(11) **EP 4 290 523 A1**
(43) Date of publication of application: **13.12.2023**
(21) Application number: 22749646.0
(22) Date of filing: 31.01.2022
(51) Int. Cl.: G16B 40/00, C12M 3/00, C12N 5/07, C12P 21/02, C12P 21/08, C12Q 1/04

(54) **ESTIMATION DEVICE, LEARNING DEVICE, OPTIMIZATION DEVICE, ESTIMATION METHOD, LEARNING METHOD, AND OPTIMIZATION METHOD**

(30) Priority: 08.02.2021 JP 2021017968
(71) Applicant: Shimadzu Corporation, Nakagyo-ku, Kyoto-shi, Kyoto 604-8511 (JP)
(72) Inventor: SUZUKI, Takashi, Kyoto-shi, Kyoto 604-8511 (JP); YAMAMOTO, Shuhei, Kyoto-shi, Kyoto 604-8511 (JP); TOYODA, Kenichi, Kyoto-shi, Kyoto 604-8511 (JP); KURODA, Hirotaka, Kyoto-shi, Kyoto 604-8511 (JP)
(74) Representative: Müller-Boré & Partner Patentanwälte PartG mbB
(86) International application number: PCT/JP2022/003522
(87) International publication number: WO 2022/168774

(57) **Abstract**

An estimation device (200) generates quality prediction data (540) indicating a quality of a drug substance of a biopharmaceutical manufactured by culturing cells, by inputting, into a prediction model (420), measurement data (510) including a measurement result obtained by measuring a substance in a culturing vessel at least one timing after a predetermined time period has passed since the cells have been seeded in a medium. The prediction model (420) is generated by executing a learning process using training data (530) that includes measurement data including a measurement result obtained by measuring a substance in a culturing vessel at a plurality of timings after seeding of the cells in the medium, and quality data obtained by analyzing a drug substance of the biopharmaceutical manufactured from the cells.

## Description

### TECHNICAL FIELD

The present disclosure relates to an estimation device, a learning device, an optimization device, an estimation method, a learning method, and an optimization method.

### BACKGROUND ART

In recent years, research on biopharmaceuticals manufactured by culturing cells has been advanced. Japanese Patent No. 4496086 (PTL 1) discloses a cell culture method for generating a target protein by culturing host cells.

Biopharmaceuticals are produced using metabolism of cells that are difficult to be artificially controlled, and drugs to be used may contain components derived from living cells. Accordingly, even if they are produced according to a defined protocol, the qualities of produced biopharmaceuticals cannot be uniformed. Typically, characteristic analysis is performed for a drug substance of a biopharmaceutical obtained by cell culture, and the quality of the drug substance is verified.

### CITATION LIST

### PATENT LITERATURE

PTL 1: Japanese Patent No. 4496086

### SUMMARY OF INVENTION

### TECHNICAL PROBLEM

In consideration also of a culture step, a purification step, and a processing step, production of biopharmaceuticals requires significantly long time period, such as several weeks to several months. A drug substance to be subjected to characteristic analysis is obtained in the purification step after the culture step is completed. That is, since no result of characteristic analysis can be obtained in the culture step, determination as to whether an objective drug substance has been obtained by culture or not will be made only after completion of the purification step.

An object of the present disclosure is to predict the quality of the drug substance obtained by progress of the culture in a stage during the culture. Furthermore, another object of the present disclosure is to optimize a condition for improving the quality of the drug substance obtained by progress of the culture in the stage during the culture.

### SOLUTION TO PROBLEM

An estimation device of the present disclosure includes: a receiving unit that receives input of measurement data; a prediction unit that generates quality prediction data indicating a quality of a drug substance, by inputting, into a prediction model, the measurement data received by the receiving unit; and an output unit that outputs the quality prediction data generated by the prediction unit. The measurement data includes a measurement result obtained by measuring at least one substance in a culturing vessel containing cells and a medium, at least one timing after a predetermined time period has passed since the cells have been seeded in the medium. The prediction model is a model for predicting the quality of the drug substance of a biopharmaceutical manufactured by culturing the cells.

A learning device of the present disclosure includes: a receiving unit that receives training data; and a model generation unit that generates a prediction model by executing a learning process using the training data received by the receiving unit. The training data includes measurement data including a measurement result obtained by measuring at least one substance in a culturing vessel containing cells and a medium at a plurality of timings after seeding of the cells in the medium, and quality data obtained by analyzing a drug substance of a biopharmaceutical manufactured from the cells. The prediction model is a model for generating quality prediction data indicating the quality of a drug substance of a biopharmaceutical manufactured from cells contained in a culturing vessel during culture of the cells, based on a measurement result obtained by measuring at least one substance in the culturing vessel during culture of the cells.

An estimation method of the present disclosure includes: a step of putting cells and a medium in a culturing vessel, and culturing the cells; a step of measuring at least one substance in the culturing vessel at least one timing after a predetermined time period has passed since the cells have been seeded in the medium; and a step of generating quality prediction data indicating a quality of a drug substance, by inputting measurement data including a measurement result obtained in the measuring step, into a prediction model; and a step of outputting the quality prediction data. The prediction model is a model for predicting the quality of the drug substance of a biopharmaceutical manufactured by culturing the cells.

A learning method of the present disclosure includes: a step of putting cells and a medium in a culturing vessel, and culturing the cells; a step of measuring at least one substance in the culturing vessel at a plurality of timings after the cells are seeded in the medium; a step of analyzing a quality of a drug substance of a biopharmaceutical manufactured from the cells; and a step of generating a prediction model by executing a learning process using training data. The training data includes measurement data including a measurement result obtained by the step of measuring, and quality data obtained by the step of analyzing. The prediction model is a model for generating quality prediction data indicating the quality of a drug substance of a biopharmaceutical manufactured from cells contained in a culturing vessel during culture of the cells, based on a measurement result obtained by measuring at least one substance in the culturing vessel during culture of the cells.

An optimization device of the present disclosure includes: a receiving unit that receives a plurality of parameter values that define a culture condition for seeding cells in a medium, and quality data obtained by analyzing a drug substance of a biopharmaceutical manufactured by culturing the cells; an estimation unit that receives the plurality of parameter values and the quality data received by the receiving unit, and estimates an optimal combination of the plurality of parameter values; and an output unit that outputs the combination of the plurality of parameter values estimated by the estimation unit.

An optimization method of the present disclosure includes: a step of putting cells and a medium in a culturing vessel, and culturing the cells under a culture condition defined by a plurality of parameter values; a step of analyzing a quality of a drug substance of a biopharmaceutical manufactured by culturing the cells; and a step of receiving the plurality of parameter values, and quality data obtained by the step of analyzing the plurality of parameter values, and estimating an optimal combination of the plurality of parameter values. The step of culturing the cells adopts the optimal combination of the plurality of parameter values estimated by the step of estimating, as a new culture condition, and cultures the cells under the new culture condition.

### ADVANTAGEOUS EFFECTS OF INVENTION

According to the present disclosure, the quality of the drug substance obtained by progress of the culture in a stage during the culture can be predicted. Alternatively, according to the present disclosure, a condition for improving the quality of the drug substance obtained by progress of the culture in the stage during the culture can be optimized. Accordingly, based on the predicted quality of the drug substance, a user can take various measures, such as reviewing the protocol in the stage of the culture step before the purification step for obtaining the drug substance, extending the culture time period, or stopping the culture. As a result, the biopharmaceutical manufacturing cost can be reduced.

### BRIEF DESCRIPTION OF DRAWINGS

Fig. 1 is a diagram schematically showing an entire configuration of a prediction system according to this Embodiment 1.
Fig. 2 is a block diagram showing a hardware configuration of an estimation device.
Fig. 3 is a block diagram showing a hardware configuration of a learning device.
Fig. 4 is a diagram for illustrating measurement timings.
Fig. 5 is a diagram showing an example of measurement data.
Fig. 6 is a block diagram showing an example of a configuration of a prediction unit.
Fig. 7 is a diagram showing an example of an output result output by an output unit.
Fig. 8 is a flowchart showing a flow of an estimation method according to this Embodiment 1.
Fig. 9 is a flowchart showing a flow of a learning method according to this Embodiment 1.
Fig. 10 is a block diagram showing a configuration of a learning device according to a modification example.
Fig. 11 is a diagram showing a modification example of a model generation unit.
Fig. 12 is a block diagram showing a configuration of an estimation device according to the modification example.
Fig. 13 is a diagram showing modification examples of prediction models.
Fig. 14 is a diagram schematically showing an entire configuration of a prediction system according to this Embodiment 2.
Fig. 15 is a block diagram showing a hardware configuration of an optimization device.
Fig. 16 is a block diagram showing an example of a configuration of the optimization device.
Fig. 17 is a flowchart showing a flow of a process of the optimization device.
Fig. 18 is a diagram showing a modification example of a prediction system according to this Embodiment 2.

### DESCRIPTION OF EMBODIMENTS

Hereinafter, embodiments of the present disclosure are described in detail with reference to the drawings. In the drawings, the same or corresponding portions are assigned the same symbols, and the description of them is not repeated.

### Embodiment 1.

### [Entire configuration of prediction system]

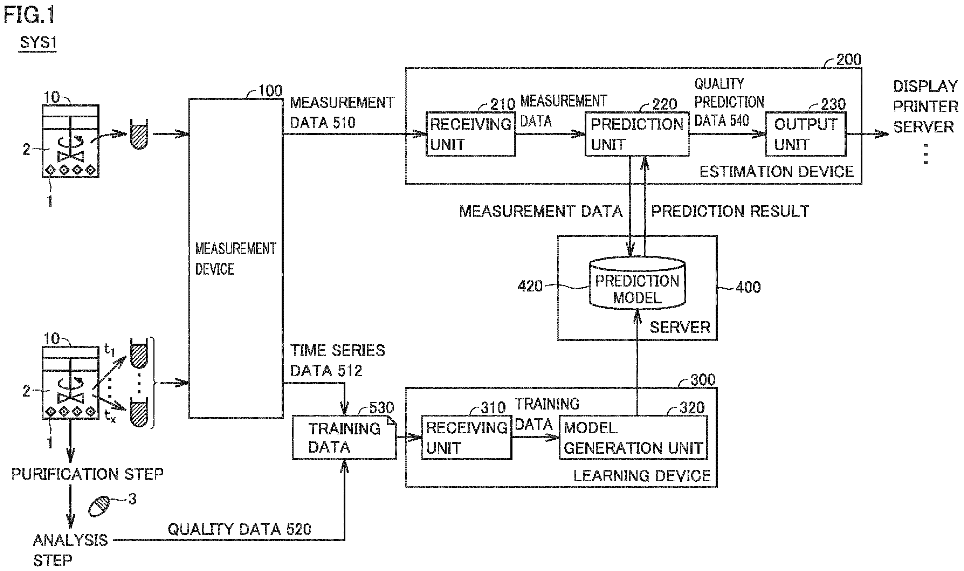

Fig. 1 is a diagram schematically showing an entire configuration of a prediction system according to this Embodiment 1. A prediction system SYS is a system for predicting the quality of a drug substance of a biopharmaceutical manufactured by cell culture. In this Embodiment 1, "biopharmaceutical" is any of pharmaceuticals manufactured using cells, e.g., antibody pharmaceuticals, and vaccines. In this Embodiment 1, biopharmaceuticals can include cells themselves used for regenerative medicine. In this Embodiment 1, "drug substance" is an objective substance obtained by cell culture, and for example, obtained by a culture step for culturing cells, and subsequently a purification step for taking out an objective component. Prediction system SYS includes a measurement device 100, an estimation device 200, a learning device 300, and a server 400.

Measurement device 100 measures a measurement target that is a substance in a culturing vessel 10 that contains cells 1, and a medium 2. Preferably, the substance as the measurement target is a substance that changes in the course of culturing cells 1, and is, for example, any of cells 1 themselves, a metabolite of cells 1, a nutrient of cells 1 and the like. The user can select any measurement device 100 in accordance with the measurement target. For example, in a case where the measurement target is cells 1, the user can select a microscope, and software for analyzing images obtained by the microscope, as measurement device 100, in order to obtain measurement results that include the number, the living ratio, and the shape (the roundness, the length of major axis, the length of minor axis, etc.) of cells in a culture solution made up of cells 1 and medium 2 in culturing vessel 10,. In a case where the measurement target is a metabolite or a nutrient of cells 1, the user can select a liquid chromatography mass spectrometer (LC-MS), ICP (inductively coupled plasma) mass spectrometer or the like, as measurement device 100, in order to obtain measurement results, such as the concentration of a metabolite and the concentration of a nutrient in the culture solution.

Estimation device 200 predicts the quality of the drug substance, using measurement data 510 that includes measurement results about the measurement target which is obtained after a predetermined time period has passed since cells 1 have been seeded in medium 2. For example, measurement data 510 is obtained by collecting the culture solution in culturing vessel 10 at least one timing after the predetermined time period has passed since cells 1 have been seeded in medium 2, and measuring a substance in the collected culture solution using measurement device 100.

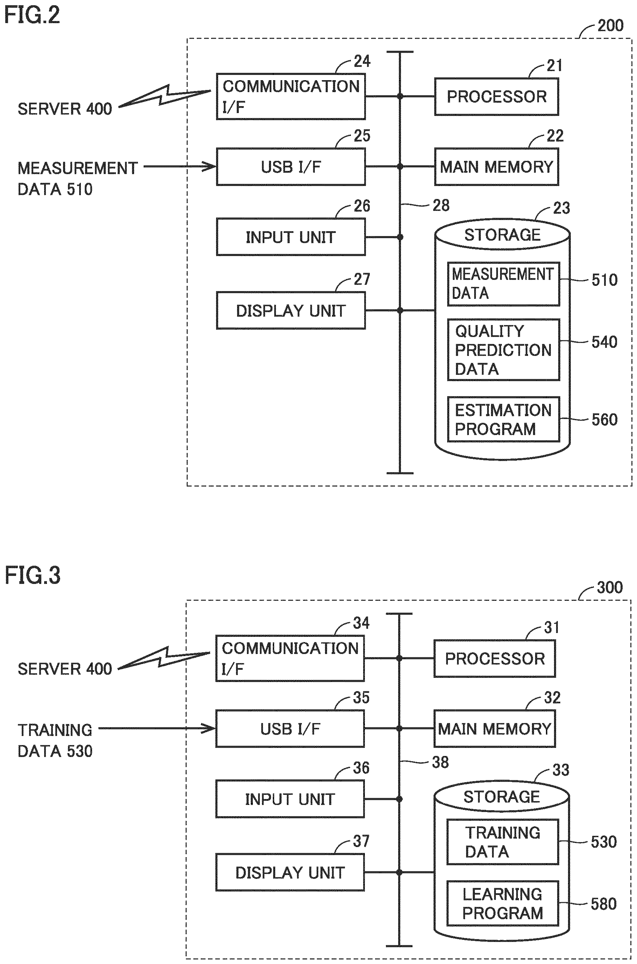

Estimation device 200 includes a receiving unit 210, a prediction unit 220, and an output unit 230, as an example of a software configuration implemented by a processor 21 (see Fig. 2) executing an estimation program 560 (see Fig. 2). Receiving unit 210 receives input of measurement data 510.

Prediction unit 220 generates quality prediction data 540 indicating the quality of the drug substance, by inputting measurement data 510 received by receiving unit 210 into a prediction model 420. Prediction model 420 is a model for predicting the quality of the drug substance of the biopharmaceutical manufactured by culturing cells 1, and outputs a prediction result indicating the quality of the drug substance upon input of measurement data 510. Prediction model 420 is generated by a model generation unit 320 of learning device 300 executing a supervised learning process.

Quality prediction data 540 may be the prediction result itself output from prediction model 420, or may be what is generated based on the prediction result output from prediction model 420. The prediction result output from prediction model 420 is a result that corresponds to quality data 520 used as correct data when model generation unit 320 is generated. For example, in a case where quality data 520 is data itself indicating an analysis result obtained by applying any analysis to a drug substance 3, prediction model 420 outputs an analysis result predicted as the prediction result. In a case where quality data 520 is data indicating the similarity to the objective drug substance obtained based on the analysis result, prediction model 420 outputs the similarity predicted as the prediction result.

Output unit 230 outputs quality prediction data 540 generated by prediction unit 220 to any output destination, such as a display, a printer, and a server.

Learning device 300 generates prediction model 420 using training data 530. Learning device 300 includes a receiving unit 3 10, and model generation unit 320, as an example of a software configuration implemented by a processor 31 (see Fig. 3) executing a learning program 580 (see Fig. 3).

Receiving unit 310 receives input of training data 530. Training data 530 includes time series data 512, and quality data 520.

Time series data 512 is a type of measurement data 510, and is obtained by measuring at least one substance in culturing vessel 10 at a plurality of timings (timing t1,..., timing tx) after seeding of cells 1 in medium 2. Time series data 512 is data indicating the temporal change of the measurement target, which is a substance in culturing vessel 10 containing cells 1 and medium 2. Time series data 512 is obtained by collecting the culture solution in culturing vessel 10 at a plurality of timings (timing t1,..., timing tx) after seeding of cells 1 in medium 2, and measuring each of portions of collected culture solution using measurement device 100. For example, time series data 512 indicates the temporal change in cell number, temporal change in living cell ratio, temporal change in shape of cells, temporal change in concentration of a nutrient, temporal change in concentration of a metabolite and the like.

Quality data 520 is obtained by analyzing drug substance 3 of a biopharmaceutical manufactured from cells 1. Quality data 520 is data obtained after culturing cells 1, and subsequently applying the purification step and an analysis step. Quality data 520 indicates the quality of drug substance 3 that is obtained by applying any analysis in the analysis step to drug substance 3 obtained through the purification step. The analysis performed in the analysis step is freely selected in accordance with the type of drug substance 3. The quality of drug substance 3 is evaluated from one or more perspectives, and for example, is evaluated from perspectives of physical property, chemical property, bioactivity, immunochemical property and the like. Quality data 520 may be data indicating an analysis result obtained by applying any analysis to drug substance 3, or data indicating the similarity to the objective drug substance obtained, based on the analysis result.

For example, in a case where drug substance 3 is utilized as an antibody pharmaceutical, amino acid composition analysis, amino acid sequence analysis, peptide mapping analysis, analysis of the cross-linked position of disulfide bonding, glycosylation structure analysis and the like are performed for drug substance 3 in the analysis step. In this case, for example, as a result of amino acid composition analysis, data indicating how much degree of similarity between the amino acid composition and the objective drug substance may be included in quality data 520.

Model generation unit 320 generates prediction model 420 by executing the learning process, with quality data 520 included in training data 530 being adopted as correct data. For example, model generation unit 320 inputs time series data 512 included in training data 530 into prediction model 420, obtains an error between the output prediction result of the quality and quality data 520 that is correct data, and optimizes prediction model 420 so as to reduce the error.

Server 400 is an example of a storage device for storing prediction model 420. Note that prediction model 420 may be stored in a storage 23 of estimation device 200 (see Fig. 2), or stored in a storage 33 of learning device 300 (see Fig. 3). Estimation device 200 and learning device 300 may be implemented by a single device. In the case where estimation device 200 and learning device 300 are implemented by a single device, receiving unit 210 and receiving unit 310 may be implemented by a single receiving unit.

Note that in this embodiment, the culture condition is assumed to be the same between a case of generating prediction model 420, and a case of generating quality prediction data 540 using generated prediction model 420. In this embodiment, the fact that "culture condition" is the same means that culture is performed according to a common protocol.

Even if cell culture is performed according to the common protocol, the drug substance obtained by the cell culture may exhibit characteristics different from those of the objective drug substance because the cells themselves are different, and a component derived from living cells is contained in a drug to be used in some cases. Accordingly, characteristic analysis is typically performed for a drug substance obtained by cell culture in order to secure the quality of biopharmaceuticals.

Drug substance 3 is obtained by activity of cells 1. Accordingly, it is expected that there is a relationship between the characteristics of drug substance 3 and the activity situations of cells 1. It is expected that the substance in culturing vessel 10 containing cells 1 exhibits different temporal changes between cases where the activities of cells 1, such as growth and metabolism, are active and inactive. Accordingly, it is expected that there is a relationship between the state of the substance in culturing vessel 10 and the activity situations of cells 1. That is, it is expected that there is indirect relationship between the state of the substance in culturing vessel 10 and the characteristics of drug substance 3.

Learning device 300 generates prediction model 420 by executing the learning process using training data 530 that includes time series data 512 and quality data 520 indicating temporal change in substance in culturing vessel 10 and the quality of drug substance 3 respectively. In other words, learning device 300 generates prediction model 420 by learning of the relationship between the temporal change in substance in culturing vessel 10 and the quality of drug substance 3.

The user repeatedly executes a culture examination that includes the culture step and the purification step, while changing the culture condition. In each culture examination, quality data 520 is obtained. Measurement device 100 outputs time series data 512 with respect to each culture examination. By repeatedly executing the culture examination, a large number of training data 530 in accordance with the number of culture examinations are collected. Learning device 300 repeatedly optimizes prediction model 420, based on the large number of training data.

In this Embodiment 1, measurement data 510 is obtained by collecting the culture solution in culturing vessel 10 at least one timing after the predetermined time period has passed since cells 1 have been seeded in medium 2, and measuring a substance in the collected culture solution using measurement device 100. That is, measurement data 510 indicates the state of the substance in the culture solution at least one timing after the predetermined time period has passed since cells 1 have been seeded in medium 2.

Measurement data 510 indicates the state of the substance after the predetermined time period has passed since cells 1 have been seeded in medium 2. Accordingly, by inputting measurement data 510 into prediction model 420 generated by learning of the relationship between the temporal change in substance and the quality of drug substance 3, a prediction result is obtained that indicates the quality of drug substance 3 obtained after progress of culture.

As described above, estimation device 200 according to this Embodiment 1 generates quality prediction data 540 indicating the quality of the drug substance obtained by progress of culture of cells 1 in culturing vessel 10, based on measurement data 510 including the measurement result obtained by measuring the measurement target in culturing vessel 10. Accordingly, the quality of the drug substance obtained by progress of the culture in a stage during the culture can be predicted. In the stage during the culture, learning device 300 can generate a prediction model for predicting the quality of the drug substance obtained by progress of the culture. Accordingly, based on quality prediction data 540, the user can take various measures, such as reviewing the protocol in the stage of the culture step before the purification step for obtaining the drug substance, extending the culture time period, and stopping the culture. As a result, the biopharmaceutical manufacturing cost can be reduced.

Learning device 300 according to this Embodiment 1 can generate prediction model 420 where the relationship between the temporal change in substance in culturing vessel 10 and the quality of drug substance 3 has been learned, by executing the learning process using training data 530 that includes time series data 512 and quality data 520 indicating the temporal change in substance in culturing vessel 10 and the quality of drug substance 3, respectively.

### [Hardware configuration of estimation device]

Fig. 2 is a block diagram showing a hardware configuration of estimation device 200. Estimation device 200 includes processor 21, a main memory 22, storage 23, a communication interface (I/F) 24, a USB (universal serial bus) interface (I/F) 25, an input unit 26, and a display unit 27. These components are connected to each other via a processor bus 28.

Processor 21 is made up of a CPU (central processing unit), a GPU (graphics processing unit) and the like, reads a program (for example estimation program 560) stored in storage 23, loads the program in main memory 22 and executes the program. Processor 21 performs a series of processes for predicting the quality of the drug substance by executing the program.

Main memory 22 is made up of, for example, a volatile storage device, such as a RAM (random access memory), or a DRAM (dynamic RAM). Storage 23 is made up of, for example, a nonvolatile storage device, such as an HDD (hard disk drive) or an SSD (solid state drive).

Storage 23 stores measurement data 510 transmitted via USB I/F 25, quality prediction data 540 generated based on measurement data 510, estimation program 560 for performing the series of processes for predicting the quality of the drug substance, and the like.

Communication I/F 24 exchanges a signal with server 400 that stores prediction model 420, using wired communication or wireless communication.

A USB memory (not shown) is detachably attached to USB I/F 25, through which measurement data 510 stored in the USB memory is read. Note that measurement data 510 may be transmitted from measurement device 100 to estimation device 200 by communicably connecting measurement device 100 and estimation device 200 to each other.

Input unit 26 accepts user operation, and is typically made up of a touch panel, a keyboard, a mouse and the like. Display unit 27 is an example of an output destination of quality prediction data 540, and is made up of a liquid crystal panel or the like that can display an image.

### [Hardware configuration of learning device]

Fig. 3 is a block diagram showing a hardware configuration of learning device 300. Learning device 300 includes processor 31, a main memory 32, storage 33, a communication I/F 34, a USB I/F 35, an input unit 36, and a display unit 37. These components are connected to each other via a processor bus 38.

Processor 31 includes a CPU and a GPU, reads a program (e.g., learning program 580) stored in storage 33, loads the program in main memory 32, and executes the program. Processor 31 performs a series of processes for generating prediction model 420 by executing the program.

Main memory 32 is made up of, for example, a volatile storage device, such as a RAM or a DRAM. Storage 33 is made up of, for example, a nonvolatile storage device, such as an HDD or an SSD.

Storage 33 stores training data 530 transmitted via USB I/F 35, and learning program 580 for performing the series of processes for generating prediction model 420.

Communication I/F 34 exchanges a signal with server 400 that stores prediction model 420, using wired communication or wireless communication.

A USB memory (not shown) is detachably attached to USB I/F 35, through which training data 530 stored in the USB memory is read. Note that training data 530 may be transmitted from an information processing device to estimation device 200, by accepting input of measurement data 510 and quality data 520, associating measurement data 510 and quality data 520 with each other, and communicably connecting the information processing device that generates training data 530 and learning device 300 to each other.

Input unit 36 accepts user operation, and is typically made up of a touch panel, a keyboard, a mouse and the like. Display unit 37 is made up of a liquid crystal panel or the like that can display an image.

### [Example of measurement data 510 utilized for quality prediction]

Measurement data 510 that estimation device 200 accepts to predict the quality of drug substance 3 includes a measurement result obtained by measuring the measurement target at least one timing after the predetermined time period has passed since cells 1 have been seeded in medium 2. Note that measurement data 510 used to predict the quality of drug substance 3 may be time series data 512 indicating the temporal change in measurement target, as a measurement result obtained by measuring the measurement target at a plurality of timings after seeding of cells 1 in medium 2.

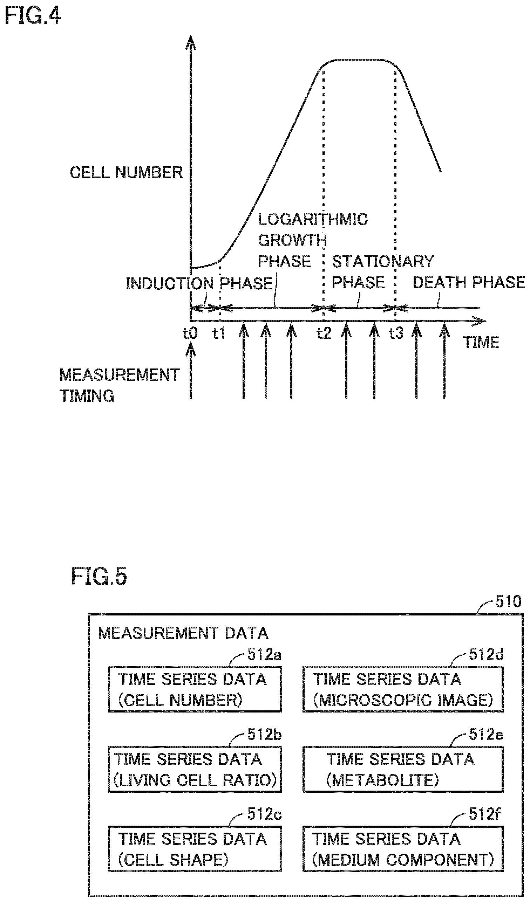

Fig. 4 is a diagram for illustrating the measurement timings. In Fig. 4, a typical growth curve is depicted. As shown in Fig. 4, typically, immediately after seeding of cells 1 in medium 2, immediate growth is not exhibited, the phase transitions to an induction phase (or also called a lag phase) and then to a growing logarithmic growth phase. Subsequently, due to reduction in nutrient or accumulation of waste (metabolite), the logarithmic growth phase is finished, and the phase transitions to a stationary phase (or also called a quiescent phase). In the stationary phase, the balance between the number of growing cells 1, and the number of extinct cells 1 is maintained. The stationary phase continues for a while, subsequently the extinct cells 1 increase, and then the phase transitions to the death phase (or also called a decline phase).

Measurement data 510 may include a measurement result obtained by measuring the measurement target at a timing t0 of starting to culture cells 1. Note that the timing of starting to culture cells 1 can include a timing before seeding cells 1 in medium 2, and a timing immediately after seeding cells 1 in medium 2. A measurement result at timing t0 indicates an initial state in culturing vessel 10. If measurement data 510 includes the measurement result indicating the initial state in culturing vessel 10, measurement data 510 indicates change from the initial state.

Measurement data 510 may include a measurement result obtained by measuring the measurement target between a timing t1 and a timing t2 which indicate a logarithmic growth phase. That is, at least one timing after a predetermined time period has passed since cells 1 have been seeded in medium 2 is a timing between timing t1 and timing t2, which indicate the logarithmic growth phase. In the logarithmic growth phase, the number of cells 1 largely varies. Accordingly, the state of the substance in culturing vessel 10 largely varies. By including, in time series data 512, the result obtained by measuring the measurement target at the timing when a large change in state occurs as described above, estimation device 200 can predict the quality of drug substance 3 using data more correctly indicating the change in state of cells 1.

Measurement data 510 may include a measurement result obtained by measuring the measurement target between timing t2 and a timing t3 which indicate the stationary phase. That is, at least one timing after a predetermined time period has passed since cells 1 have been seeded in medium 2 is a timing between timing t2 and timing t3, which indicate the stationary phase. In the stationary phase, the balance between the number of growing cells 1, and the number of extinct cells 1 is maintained, and the number of cells 1 becomes the maximum. By including, in time series data 512, the result obtained by measuring the measurement target at the timing when the number of cells 1 becomes the maximum as described above, estimation device 200 can predict the quality of drug substance 3 using data indicating the state of the substance in culturing vessel 10 when the number of cells 1 becomes the maximum.

Measurement data 510 (time series data 512) includes a measurement result measured in the death phase after timing t3. Since the cells become extinct, enzymes and the like in the cells are exposed into the medium, which possibly affects the quality of the drug substance. In this case, the measurement result measured in the death phase of the cells can be possibly utilized for machine learning as an example of data of an inferior quality.

Fig. 5 is a diagram showing an example of measurement data 510. Note that in an example shown in Fig. 5, time series data 512a to 512f each obtained by measurement at a plurality of timings after seeding of cells 1 in medium 2 are exemplified. However, each of time series data 512a to 512f may be a data item including a measurement result obtained by measuring the measurement target at least one timing after a predetermined time period has passed since cells 1 have been seeded in medium 2.

Measurement data 510 includes, for example, time series data item 512a on the cell number, time series data item 512b on the living cell ratio, time series data item 512c on the cell shape, time series data item 512d on an microscopic image or the like, as the measurement results obtained by measuring cells 1.

Measurement data 510 includes, for example, time series data item 512a on the metabolite, as a measurement result obtained by measuring the metabolite generated by metabolism of cells 1. Note that the metabolite includes at least one of a metabolite secreted by cells 1 to the outside, and a metabolite contained in cells 1 themselves.

Furthermore, measurement data 510 includes, for example, time series data item 512f on the medium component, as a measurement result obtained by measuring a nutrient to be ingested by cells 1. Note that in a case where cells 1 generate a component common to a component contained in the medium by metabolism, time series data item 512f on the medium component can serve as the time series data item on the metabolism.

That is, a substance as a measurement target is at least one of a nutrient to be ingested by cells 1, a metabolite generated by metabolism of cells 1, and cells 1. Note that measurement data 510 may include information on the pH, temperature and the like of the culture solution in culturing vessel 10.

Referring to Figs. 4 and 5, measurement data 510 accepted by estimation device 200 has been described. Similar to measurement data 510 accepted by estimation device 200, time series data 512 included in training data 530 accepted by learning device 300 may also include multiple types of time series data.

For example, time series data 512 included in training data 530 may indicate the temporal change in the measurement target, with at least one substance among the nutrient ingested by cells 1, the metabolite generated by metabolism of cells 1, and cells 1 being adopted as the measurement target.

More specifically, time series data 512 included in training data 530 includes, for example, a time series data item on the cell number, a time series data item on the living cell ratio, a time series data item on the cell shape, a time series data item on an microscopic image and the like, which are obtained by measuring the cells. Time series data 512 included in training data 530 includes a time series data item on the metabolite obtained by measuring the metabolite. Time series data 512 included in training data 530 includes a time series data item on the medium component obtained by measuring the nutrient. Note that time series data 512 included in training data 530 can include information on the pH, temperature and the like of the culture solution in culturing vessel 10.

Time series data 512 used when prediction model 420 is generated, and measurement data 510 input into prediction model 420 when quality prediction data 540 is generated using prediction model 420 include at least a measurement result obtained by adopting a mutually common substance as the measurement target. That is, if time series data indicating the temporal change in cell number is used when prediction model 420 is generated, measurement data 510 input into prediction model 420 includes at least a measurement result indicating the cell number.

### [Configuration of prediction unit 220 of estimation device 200]

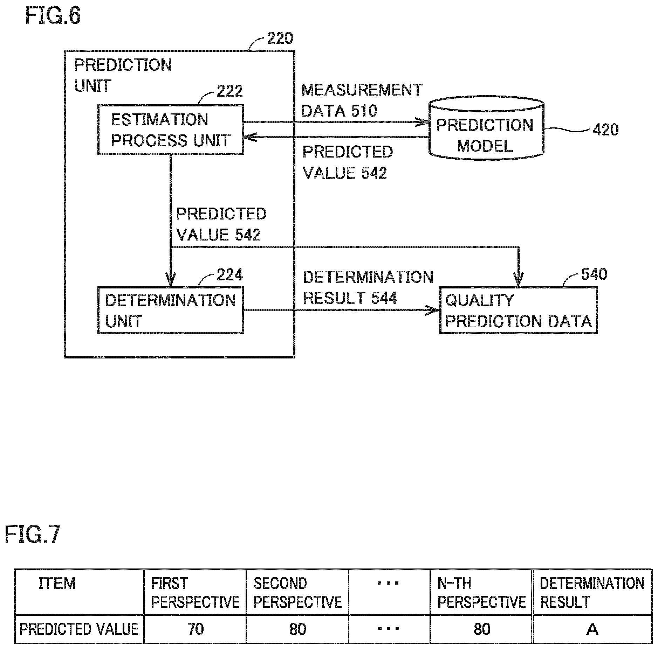

Fig. 6 is a block diagram showing an example of a configuration of prediction unit 220. Prediction unit 220 includes an estimation process unit 222, and a determination unit 224. Estimation process unit 222 inputs measurement data 510 received by receiving unit 210, into prediction model 420, and obtains a prediction result. The prediction result may be, for example, a predicted value 542.

Typically, the quality of drug substance 3 is evaluated from one or more perspectives. Accordingly, prediction model 420 may be configured to output, as the prediction result, predicted value 542 evaluated from a specific perspective. Predicted value 542 is, for example, an estimate of a result obtained when drug substance 3 obtained from cells in culture are analyzed in a specific perspective, or a similarity indicating how much degree the obtained estimate is similar to the result of the objective drug substance.

Perspectives for evaluating the quality of drug substance 3 include, for example, the physical property, chemical property, bioactivity, immunochemical property and the like. Prediction model 420 may be configured to output a plurality of predicted values obtained by evaluating drug substance 3 from the respective perspectives. For example, prediction model 420 may output a first predicted value obtained in the case of evaluation from a first perspective (e.g., the physical property), and a second predicted value obtained in the case of evaluation from a second perspective (e.g., bioactivity).

Note that prediction model 420 may be configured to include a first prediction model for outputting the first predicted value in the case of evaluation of the quality of the drug substance from the first perspective, and a second prediction model for outputting the second predicted value in the case of evaluation of the quality of the drug substance from the second perspective.

Determination unit 224 determines the quality of drug substance 3, based on predicted value 542, and generates a determination result 544. Determination result 544 may be a two-stage evaluation result (e.g., good/bad), a multiple-stage evaluation result (e.g., A, B, C, ..., etc.), or a point-system evaluation result (e.g., 50 points, 95 points, ..., etc.).

In a case where prediction model 420 is configured to output a plurality of predicted values obtained when drug substance 3 is evaluated from the respective perspectives, determination unit 224 determines the quality of drug substance 3 from the plurality of predicted values, and generates determination result 544. Note that in this case, determination unit 224 may weight the plurality of predicted values in accordance with the degrees of importance of the respective perspectives.

Quality prediction data 540 includes predicted value 542 generated by estimation process unit 222, and determination result 544 generated by determination unit 224. For example, in the case where prediction model 420 is configured to output a plurality of predicted values obtained when drug substance 3 is evaluated from the respective perspectives, quality prediction data 540 includes the plurality of predicted values (the first predicted value, second predicted value, etc.).

By including, in quality prediction data 540, the plurality of predicted values obtained when drug substance 3 is evaluated at the respective perspectives, the quality of the drug substance can be evaluated from various perspectives.

Since quality prediction data 540 includes determination result 544, the prediction result for the quality of the drug substance can be presented in a more easily understandable manner.

Output unit 230 outputs quality prediction data 540. Fig. 7 is a diagram showing an example of an output result output by output unit 230.

As shown in Fig. 7, output unit 230 may output determination result 544 in combination with predicted value 542 obtained with respect to each perspective.

Note that prediction unit 220 is not necessarily include determination unit 224 that comprehensively determines the quality of the drug substance.

### [Estimation method]

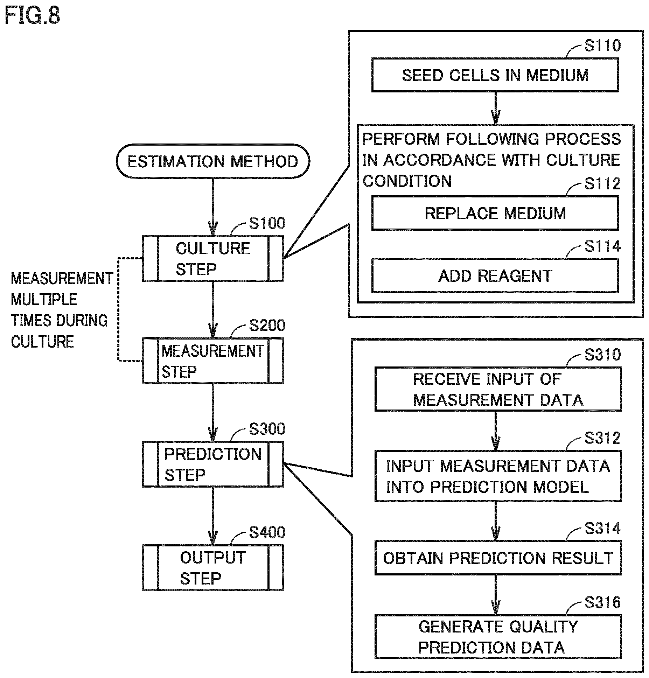

Fig. 8 is a flowchart showing a flow of an estimation method according to this Embodiment 1. The estimation method includes: a culture step (S100) of culturing cells; a measurement step (S200) of measuring the substance in the culturing vessel; a prediction step (S300) of predicting the quality of drug substance 3, based on measurement data 510 that includes a measurement result; and an output step (S400) of outputting quality prediction data 540 obtained in the prediction step.

Culture step (S100) includes a step (S110) of seeding cells 1 in medium 2. Culture step (S100) can include a step (S112) of replacing medium 2 according to the culture condition (predefined protocol), and a step (S114) of adding a reagent. Note that medium 2 may be a liquid or a solid.

Measurement step (S200) can be performed once or multiple times in culture step (S100). Measurement step (S200) is performed at any timing, for example, the timing when step (S110) of seeding cells 1 in medium 2 is performed, the timing when step (S112) of replacing medium 2 is performed, the timing when step (S114) of adding a reagent is performed, etc.

That is, the measurement timing may be any of the timing when step (S110) of seeding cells 1 in medium 2 is performed, the timing when step (S112) of replacing medium 2 is performed, the timing when step (S114) of adding a reagent is performed, etc. By matching the operation timing occurring in culture step (S100) with the measurement timing as described above, contamination can be prevented. Note that as described with reference to Fig. 4, the measurement timing may be in the logarithmic growth phase, in the stationary phase, or in the death phase.

By performing measurement step (S200) once or multiple times in culture step (S100), the substance in culturing vessel 10 is measured at least one timing after the predetermined time period has passed since cells 1 have been seeded in medium 2, and a measurement result is obtained.

Note that culture step (S100) and measurement step (S200) may be performed manually, or automated or semi-automated by a machine.

Prediction step (S300) includes a step (S310) of accepting input of measurement data 510; a step (S312) of inputting measurement data 510 into prediction model 420; a step (S314) of obtaining a prediction result from prediction model 420; and a step (S316) of generating quality prediction data 540. That is, in prediction step (S300), by inputting, into prediction model 420, measurement data 510 that includes the measurement result obtained in measurement step (S200), quality prediction data 540 can be generated. Specifically, S310 to S316 and S400 are performed by estimation device 200.

### [Learning method]

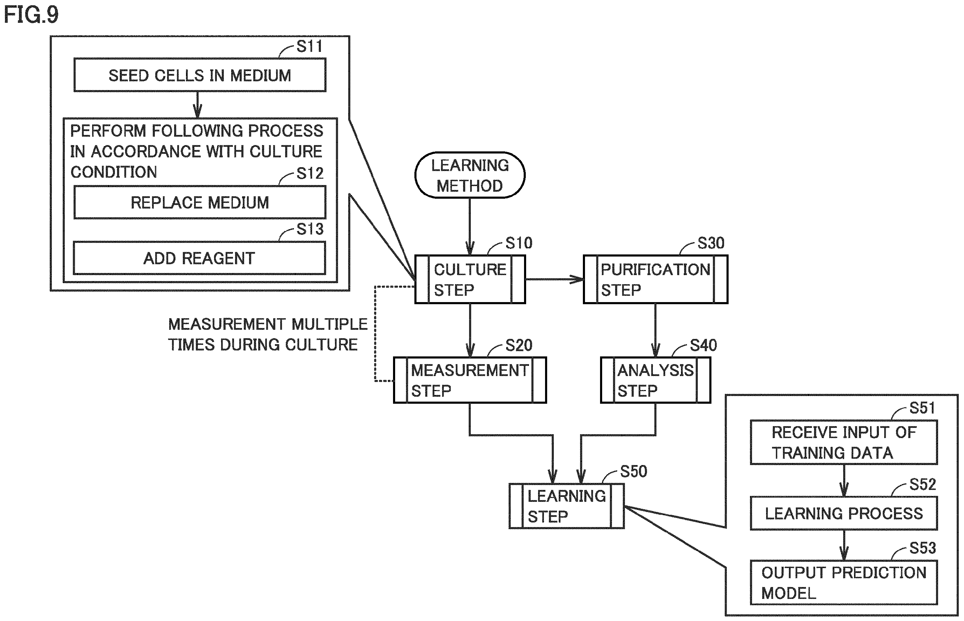

Fig. 9 is a flowchart showing the flow of the learning method according to this Embodiment 1. The learning method includes: a culture step (S10) of culturing cells; a measurement step (S20) of measuring a substance in the culturing vessel; a purification step (S30) of obtaining a drug substance; an analysis step (S40) of analyzing the quality of the drug substance; and a learning step (S550) of executing the learning process using training data 530, and generating prediction model 420.

Culture step (S10) is in common with culture step (S100) included in the estimation method. Specifically, culture step (S1 0) includes a step (S11) of seeding cells 1 in medium 2. Culture step (S10) can include a step (S12) of replacing medium 2 according to the culture condition (predefined protocol), and a step (S13) of adding a reagent. Note that medium 2 may be a liquid or a solid.

Measurement step (S20) can be performed multiple times in culture step (S10). Accordingly, time series data 512 indicating the temporal change in measurement target is obtained. Measurement step (S200) is performed at any timing, for example, the timing when step (S11) of seeding cells 1 in medium 2 is performed, the timing when step (S12) of replacing medium 2 is performed, the timing when step (S13) of adding a reagent is performed, etc.

That is, the measurement timing may be any of the timing when step (S 11) of seeding cells 1 in medium 2 is performed, the timing when step (S112) of replacing medium 2 is performed, the timing when step (S114) of adding a reagent is performed, etc. By matching the operation timing occurring in culture step (S100) with the measurement timing as described above, contamination can be prevented. Note that as described with reference to Fig. 4, the measurement timing may be in the logarithmic growth phase, in the stationary phase, or in the death phase.

By performing measurement step (S200) multiple times in culture step (S 100), the substance in culturing vessel 10 is measured at a plurality of timings after seeding of cells 1 in medium 2, and a measurement result is obtained.

Note that culture step (S10) and measurement step (S20) may be performed manually, or automated or semi-automated by a machine.

Purification step (S30) is operation of extracting only an objective component from the culture solution in culturing vessel 10. Through purification step (S30), the drug substance that contains the objective component of high purity can be obtained.

In analysis step (S40), the drug substance is evaluated from one or more perspectives. Thus, one or more types of analyses are performed, and quality data 520 is obtained. In the case where the multiple types of analyses are performed, quality data 520 can include multiple types of analysis results (analytical results).

Learning step (S50) includes: a step (S51) of accepting input of training data 530; a step (S52) of performing the learning process using training data 530, and a step (S53) of outputting prediction model 420 generated by the learning process. Note that in this Embodiment 1, the output destination of prediction model 420 is server 400. Alternatively, prediction model 420 may be stored in storage 33 of learning device 300 without being output. Specifically, S51 to S53 are performed by learning device 300.

### <Modification example>

### [Modification example of learning device]

In Embodiment 1 described above, learning device 300 is assumed to obtain training data 530 from the outside. Note that the learning device may have a function of generating training data 530. In Embodiment 1 described above, referring to Fig. 4, the measurement timing for obtaining measurement data 510 accepted by estimation device 200 is described. The growth curve of cells 1 varies depending on the type of cells 1. Accordingly, the learning device may have a function for identifying the measurement timing in prediction for generating quality prediction data 540.

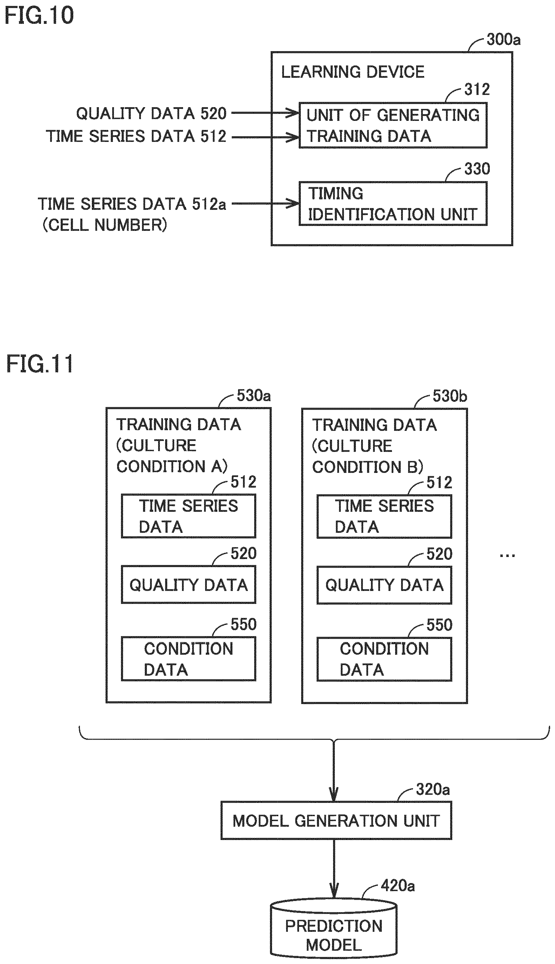

Fig. 10 is a block diagram showing the configuration of a learning device according to a modification example. Fig. 10 shows a configuration that learning device 300 according to Embodiment 1 described above does not include. A learning device 300a is different from learning device 300 in that learning device 300a further includes a unit 312 of generating training data, and a timing identification unit 330.

Unit 312 of generating training data generates training data 530, based on time series data 512 and quality data 520. Although not shown, unit 312 of generating training data transmits generated training data 530 to receiving unit 310. Receiving unit 310 receives input of transmitted training data 530.

Timing identification unit 330 identifies the measurement timing in prediction for generating quality prediction data 540, based on time series data item 512a on the cell number. More specifically, timing identification unit 330 identifies the induction phase, logarithmic growth phase, stationary phase, and death phase (see Fig. 4), based on time series data item 512a, and generates information that can identify timing t1 to timing t3. The information that can identify timing t1 to timing t3 includes, for example, a time period after seeding of cells 1 in medium 2. The user can determine the measurement timing in prediction, based on information that can identify timing t1 to timing t3.

### [Culture condition]

Note that in Embodiment 1 described above, the culture condition is assumed to be the same between a case of generating prediction model 420, and a case of generating quality prediction data 540 using generated prediction model 420. Note that the culture condition is not necessarily common between the case of generating prediction model 420, and the case of generating quality prediction data 540 using generated prediction model 420. The learning method and the estimation method in this case are described with reference to Figs. 11 to 13.

Fig. 11 is a diagram showing a modification example of a model generation unit. A model generation unit 320a according to the modification example generates a prediction model 420a, based on multiple types of training data 530a and 530b.

Each of training data 530a and 530b includes condition data 550. Condition data 550 is information indicating the culture condition. For example, by repeating the culture step, measurement step, purification step, and analysis step, while changing the culture condition (see Fig. 9), training data 530a and 530b generated under culture conditions different from each other are obtained.

The difference of the culture condition can include the difference in type of drug to be used, such as the difference in component of medium 2, and the difference in type of reagent to be added, and can further include the difference in temperature condition, the difference in timing of adding the reagent, etc.

Condition data 550 can include information on the drug to be used, the temperature condition, and information on the timing of adding the reagent. Note that condition data 550 is not necessarily information on a specific culture technique, and may be information that only indicates that training data 530a and 530b have been obtained in culture conditions (e.g., a condition A, a condition B, etc.) different from each other.

Model generation unit 320a generates prediction model 420a through learning of the relationship between the temporal change of the substance in culturing vessel 10 and the quality of drug substance 3, and further through the relationship between the difference in culture condition and the temporal change of the substance in culturing vessel 10, or the relationship between the difference in culture condition and the quality of drug substance 3.

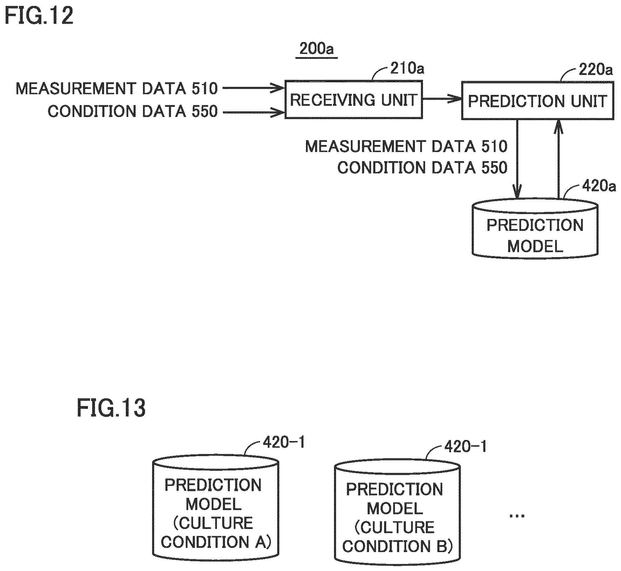

Fig. 12 is a block diagram showing the configuration of an estimation device according to the modification example. In Fig. 12, illustration of the configuration of an output unit according to Embodiment 1 described above is omitted. An estimation device 200a is different from estimation device 200 according to Embodiment 1 described above in that estimation device 200a includes a receiving unit 210a instead of receiving unit 210, and includes prediction unit 220a instead of prediction unit 220.

Receiving unit 210a receives measurement data 510, and additionally receives input of condition data 550 indicating the culture condition. Prediction unit 220a inputs condition data 550 in addition to measurement data 510 into prediction model 420a generated by model generation unit 320a shown in Fig. 13, and obtains a prediction result indicating the quality of the drug substance.

Prediction model 420a is a model in consideration of the relationship between the difference in culture condition and the temporal change of the substance in culturing vessel 10, or the relationship between the difference in culture condition and the quality of drug substance 3. Accordingly prediction model 420a can more correctly predict the quality of drug substance 3 than prediction model 420 can. Even in a case where the culture condition is different between generation of prediction model 420a, and generation of quality prediction data 540 using generated prediction model 420a, prediction unit 220a can obtain the prediction result indicating the quality of the drug substance by inputting condition data 550 in addition to measurement data 510 into prediction model 420a because prediction model 420a is a model in consideration of the difference in culture condition. That is, prediction unit 220a can obtain the prediction result in consideration of the relationship between the difference in culture condition and the quality.

Fig. 13 is a diagram showing modification examples of prediction models. For example, as shown in Fig. 13, the model generation unit may generate prediction models for the respective culture conditions, for example, a prediction model 420-1 dedicated for a culture condition A, and a prediction model 420-2 dedicated for a culture condition B.

In this case, the estimation device may use prediction models in conformity respectively with the culture conditions, based on condition data 550.

### Embodiment 2.

### [Entire configuration of prediction system]

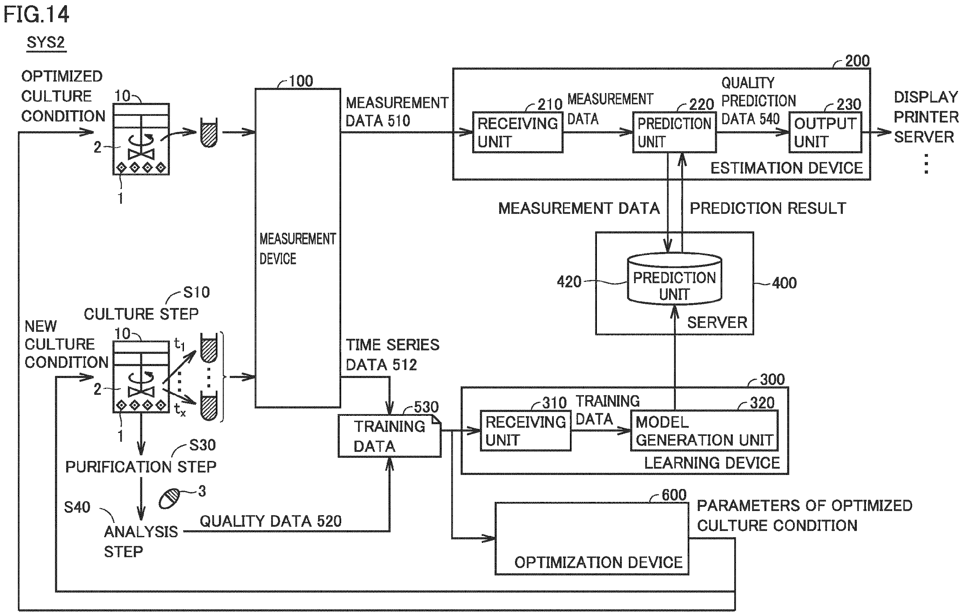

Fig. 14 is a diagram schematically showing an entire configuration of a prediction system according to this Embodiment 2. Similar to prediction system SYS1, a prediction system SYS2 has a function of predicting the quality of a drug substance of a biopharmaceutical manufactured by cell culture. Prediction system SYS2 has a function of optimizing the culture condition in addition to the functions that prediction system SYS1 has. Prediction system SYS2 includes an optimization device 600 that optimizes the culture condition.

Optimization device 600 estimates an optimal culture condition to obtain drug substance 3 of high quality, based on time series data 512 and on quality data 520, which are included in training data 530. Optimization device 600 outputs the estimated optimal culture condition. The user executes a new culture examination, based on the culture condition output from optimization device 600. Culture examination includes a culture step (step S10), a purification step (step S30), and an analysis step (step S40). These steps are similar to the respective steps described in Embodiment 1. The details of each step are shown in Fig. 9. Accordingly, the description is not repeated here.

When the culture examination is newly executed, new training data 530 is input into optimization device 600. Optimization device 600 re-estimates the optimal culture condition using training data 530 having already been input, and newly input training data 530. Optimization device 600 outputs the newly estimated culture condition. The user executes the next culture examination, based on the culture condition output from optimization device 600.

Consequently, as the number of culture examinations increases, the culture condition is further optimized. By advancing the optimization of the culture condition, the property of training data 530 is changed in a direction toward generation of drug substance 3 of higher quality. In other words, by advancing the optimization of the culture condition, the quality of training data 530 is improved. By improving the quality of training data 530, the accuracy of optimizing prediction model 420 is improved.

Optimization device 600 outputs the optimized culture condition in response to an instruction by the user. When the user predicts the quality of the drug substance using estimation device 200, the user can culture cells, based on the culture condition output from optimization device 600. Consequently, the user can predict the quality of the drug substance in an environment allowing a drug substance of high quality to be generated.

Consequently, optimization device 600 functions significantly effectively in both the processes that are the process of generating prediction model 420, and the process of predicting the quality of the drug substance by utilizing prediction model 420. According to prediction system SYS2 of Embodiment 2, the possibility of effectively obtaining the drug substance of higher quality is improved.

### [Hardware configuration of optimization device]

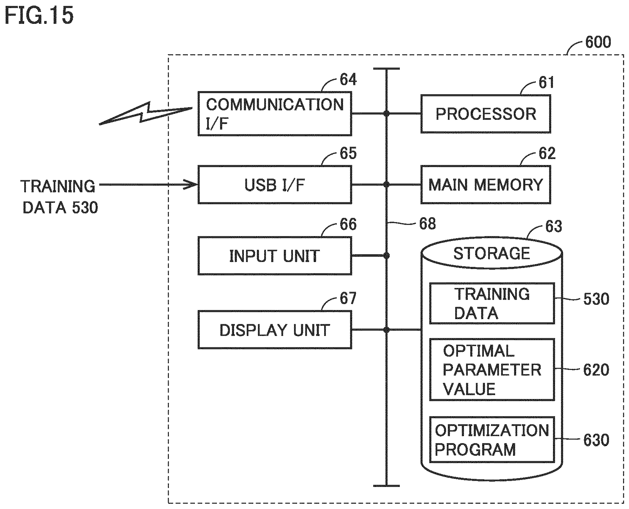

Fig. 15 is a block diagram showing the hardware configuration of optimization device 600. Optimization device 600 includes a processor 61, a main memory 62, a storage 63, a communication I/F 64, a USB I/F 65, an input unit 66, and a display unit 67. These components are connected to each other via a processor bus 68.

Processor 61 includes a CPU and a GPU, reads a program (e.g., optimization program 630) stored in storage 63, loads the program in main memory 62, and executes the program. Processor 61 performs a series of processes for retrieving the optimal parameter values of the culture condition by executing the program.

Main memory 62 is made up of, for example, a volatile storage device, such as a RAM or a DRAM. Storage 63 is made up of, for example, a nonvolatile storage device, such as an HDD or an SSD.

Storage 63 stores training data (estimation) 530 transmitted via USB I/F 65, an optimization program 630 for retrieving optimal parameter values 620 of the culture condition, and optimal parameter values 620 of the culture condition newly determined by processor 61.

Communication I/F 64 exchanges a signal with another communication device using wired communication or wireless communication.

A USB memory (not shown) is detachably attached to USB I/F 65, through which training data 530 stored in the USB memory is read.

Input unit 66 accepts user operation, and is typically made up of a touch panel, a keyboard, a mouse and the like. Display unit 67 is made up of a liquid crystal panel or the like that can display an image.

### [Configuration of optimization device 600]

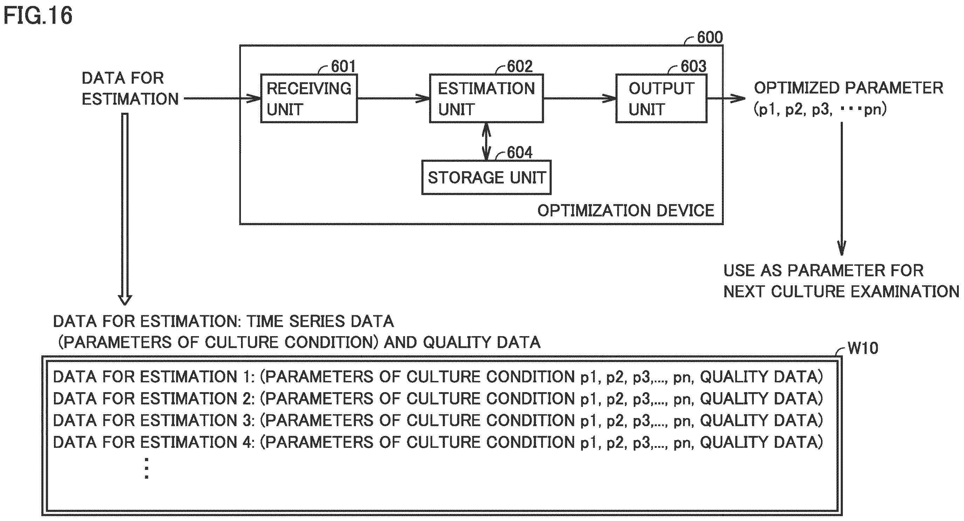

Fig. 16 is a block diagram showing an example of the configuration of optimization device 600. Optimization device 600 includes a receiving unit 601, an estimation unit 602, an output unit 603, and a storage unit 604, as an example of a software configuration implemented by processor 61 (see Fig. 15) executing optimization program 630 (see Fig. 15).

Optimization device 600 retrieves the culture condition improving the level of the quality of drug substance 3 (hereinafter also called an optimal solution) according to the Bayesian optimization. Here, the culture condition is a combination of plurality of parameter values. The Bayesian optimization is a method of estimating the optimal event from among observed events by a statistical approach, based on a Bayesian probability. According to the Bayesian optimization, a trial based on a set optimal solution is executed, and another optimal solution is retrieved based on the result of the trial. The retrieved optimal solution is set as an optimal solution used for the next trial.

Receiving unit 601 receives data for estimation. The data for estimation is included in training data 530 shown in Fig. 14. The data for estimation includes at least part of time series data 512, and quality data 520. Estimation unit 602 retrieves an optimal solution of the culture condition according to the Bayesian optimization, by executing a program for estimation. Storage unit 604 includes a plurality of memories that store the program for estimation, the input data for estimation, and the retrieved optimal solution. Output unit 603 is, for example, display unit 67 (see Fig. 15) that displays the optimal solution of the culture condition. Output unit 603 may be an interface for outputting the optimal solution of the culture condition to a display or a printer.

As shown in a window W10, one data item for estimation includes data on the culture condition and quality data used in one culture examination. The data on the culture condition includes a plurality of parameters p1, p2, ..., pn that define the culture condition. Various parameters are, for example, any of the nutrient concentration, PH, temperature, humidity, medium component, type of a reagent to be added, reagent adding timing and the like.

Various parameters are, for example, the medium condition collected at timing of seeding cells in the medium. The timing corresponds to measurement timing t0 shown in Fig. 4. That is, the values of various parameters are the parameter values measured at measurement timing t0 in time series data 512 shown in Fig. 14. Note that various parameter values measured at various measurement timings shown in Fig. 4 may be further input into receiving unit 601. For example, values that represent the temporal change in nutrient concentration, the temporal change in metabolite concentration, the change in pH of the culture solution in the culturing vessel, the change in temperature, the change in humidity and the like may be input as the data for estimation into receiving unit 601. Estimation unit 602 may retrieve the optimal culture condition, based on these data for estimation.

### [Culture condition optimization method]

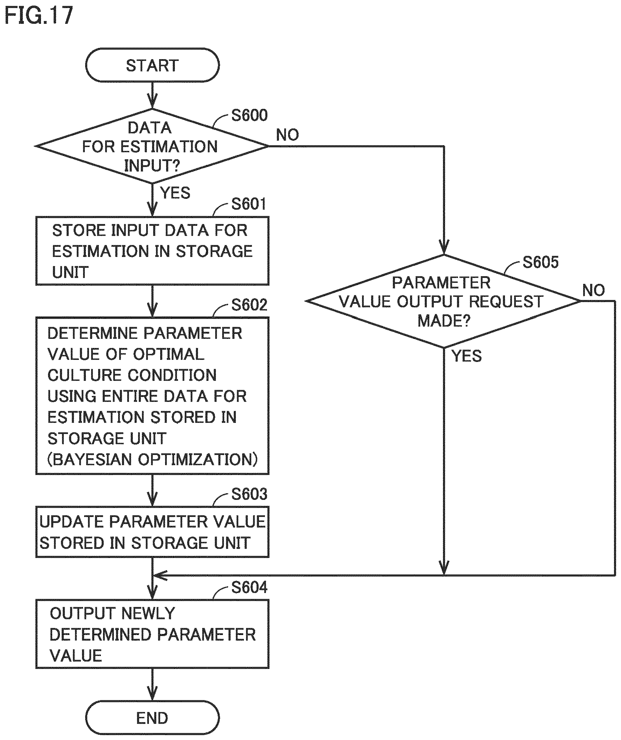

Fig. 17 is a flowchart showing the flow of a process of optimization device 600. Hereinafter, referring to Figs. 16 and 17, the flow of process of estimating the optimal culture condition by optimization device 600 is described.

First, estimation unit 602 determines whether the data for estimation is input into receiving unit 601 or not (step S600). If the data for estimation is input into receiving unit 601, estimation unit 602 stores the input data for estimation in storage unit 604 (step S601). By repetitively executing step S601, the data for estimation input into receiving unit 601 is accumulated in storage unit 604. Next, estimation unit 602 determines the values of parameters p1, p2, ..., pn of the optimal culture condition, using the entire data for estimation stored in storage unit 604 (step S602). In step S602, estimation unit 602 retrieves the culture condition providing the optimal value for the level of the quality of drug substance 3, according to Bayesian optimization.

It is desired that the retrieval of the culture condition be executed at the timing of input of a plurality of data for estimation corresponding to the respective culture examinations, in comparison with the case of execution every time input of one data item for estimation is executed. For example, it can be conceivable that the process in step S602 is executed every time of input of four to ten data for estimation that respectively correspond to four to ten culture examinations.

Next, estimation unit 602 stores the newly determined optimal parameter values in storage unit 604. If the optimal parameter values determined by the last retrieval are stored in storage unit 604, estimation unit 602 updates the values with the newly determined optimal parameter values (step S603). Next, estimation unit 602 outputs the newly determined parameter values from output unit 603 (step S604).

The parameter values output from output unit 603 is the latest parameter values indicating the optimal culture condition. The user executes a new culture examination once or multiple times based on the output parameter values. When the data for estimation corresponding to the new culture examination is input into optimization device 600, estimation unit 602 retrieves the parameter values of the optimal culture condition again. The result of retrieval is stored, as the optimal parameter values at the present time, in storage unit 604. Consequently, by repeating the culture examination and the optimization of the culture condition by optimization device 600, the optimization of the culture condition parameter values stored in optimization device 600 proceeds.

If estimation unit 602 determines NO in step S600, estimation unit 602 determines whether a request for outputting the parameter values has been made or not (step S605). For example, if the user intends to predict, through estimation device 200, the quality of the drug substance obtained by a certain culture examination, the user is required to determine the culture condition of the culture examination. It is useful to refer to the parameter values stored in optimization device 600 when the culture condition of the culture examination is determined. If it is determined in step S600 that a request for outputting the parameter values has been made, estimation unit 602 outputs the parameter values stored in storage unit 604 from output unit 603 (step S604), and finishes the process based on this flowchart.

### [Modification example of prediction system]

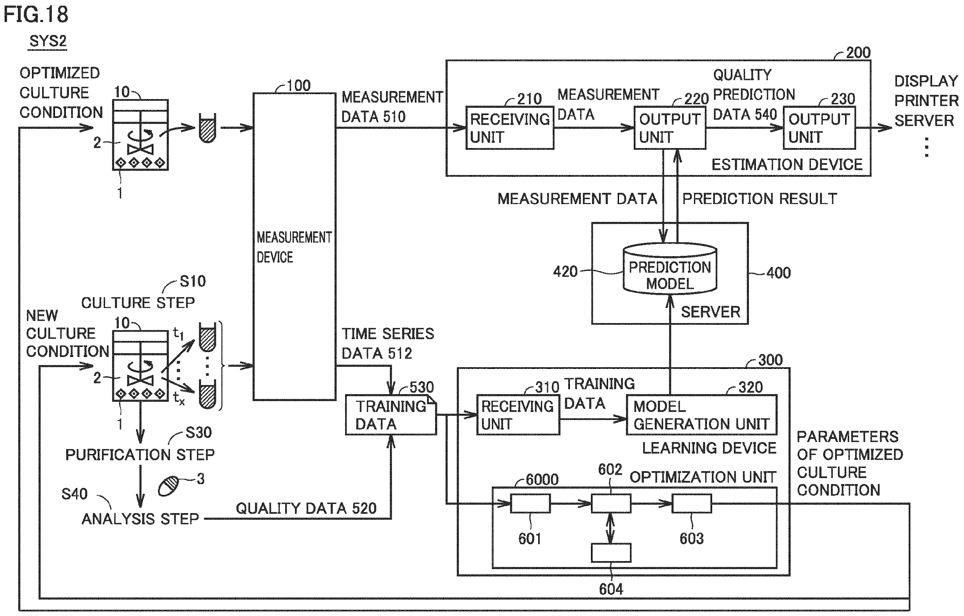

Fig. 18 is a diagram showing a modification example of a prediction system according to this Embodiment 2. In the modification example, learning device 300 includes an optimization unit 6000 that has the function of optimization device 600 shown in Fig. 16. Similar to optimization device 600, optimization unit 6000 includes receiving unit 601, estimation unit 602, output unit 603, and storage unit 604. Learning device 300 optimizes prediction model 420, based on training data 530, and retrieves the optimal parameter values of the culture condition. Consequently, according to the modification example, the added value of learning device 300 can be improved. Furthermore, according to the modification example, no optimization device 600 is required to be provided besides learning device 300. Accordingly, reduction in cost can be facilitated.

Note that optimization device 600 or optimization unit 6000 may optimize the culture condition parameter values using a grid search method instead of the Bayesian optimization.

### [Aspects]

The fact that the aforementioned embodiments are specific examples of the following aspects is understood by those skilled in the art.

(First item) An estimation device according to an aspect includes: a receiving unit that receives input of measurement data; a prediction unit that generates quality prediction data indicating a quality of a drug substance, by inputting, into a prediction model, measurement data received by the receiving unit; and an output unit that outputs the quality prediction data generated by the prediction unit. The measurement data includes a measurement result obtained by measuring at least one substance in a culturing vessel containing cells and a medium, at least one timing after a predetermined time period has passed since the cells have been seeded in the medium. The prediction model is a model for predicting the quality of the drug substance of a biopharmaceutical manufactured by culturing the cells.

In the estimation device according to the first item, the quality of the drug substance obtained by progress of the culture in a stage during the culture can be predicted. Accordingly, based on the predicted quality of the drug substance, a user can take various measures, such as reviewing the protocol in the stage of the culture step before the purification step for obtaining the drug substance, extending the culture time period, or stopping the culture. As a result, the biopharmaceutical manufacturing cost can be reduced.

(Second item) In the estimation device according to the first item, the measurement data includes a measurement result obtained by measuring the at least one substance at a plurality of timings after seeding of the cells in the medium.

(Third item) The estimation device according to item 1 or 2, wherein the measurement data includes a measurement result obtained by measuring the at least one substance at a start of culturing the cells.

(Fourth item) The estimation device according to any one of items 1 to 3, wherein the measurement data includes a measurement result obtained by measuring the at least one substance in a logarithmic growth phase of the cells.

In the logarithmic growth phase, the number of cells largely varies. Accordingly, the state of the substance in the culturing vessel is expected to largely vary. The result obtained by measuring the measurement target at the timing when a large change in state occurs is included in the measurement data. Accordingly, the estimation device according to the fourth item can predict the quality of drug substance using data more correctly indicating the change in state of cells.

(Fifth item) The estimation device according to any one of items 1 to 4, wherein the measurement data includes a measurement result obtained by measuring the at least one substance in a stationary phase of the cells.

In the stationary phase, the balance between the number of growing cells, and the number of extinct cells is maintained, and the number of cells becomes the maximum. The result obtained by measuring the measurement target at the timing when the number of cells becomes the maximum is included in the measurement data. Accordingly, the estimation device according to the fifth item can predict the quality of drug substance using data indicating the state of the substance in the culturing vessel when the number of cells becomes the maximum.

(Sixth item) The estimation device according to any one of items 1 to 4, wherein the measurement data includes a measurement result obtained by measuring the at least one substance in a death phase of the cells (Fig. 5).

In the death phase, the cells become extinct, and enzymes and the like in the cells are exposed into the medium accordingly, which possibly affects the quality of the drug substance. In this case, the measurement result measured in the death phase of the cells can be possibly utilized for machine learning as an example of data of an inferior quality.

(Seventh item) The estimation device according to any one of items 1 to 6, wherein the at least one substance is at least one of a nutrient ingested by the cell, a metabolite generated by metabolism of the cells, and the cells.

(Eighth item) The estimation device according to any one of items 1 to 7, wherein a first predicted value in a case where the quality of the drug substance is evaluated from a first perspective, and a second predicted value in a case where the quality of the drug substance is evaluated from a second perspective are output from the prediction model, by input of the measurement data received by the receiving unit. The quality prediction data includes the first predicted value, and the second predicted value.

In the estimation device according to the eighth item, the quality of the drug substance can be evaluated from various perspectives.

(Ninth item) The estimation device according to any one of items 1 to 7, wherein a predicted value in a case where the quality of the drug substance is evaluated from a predetermined perspective is output from the prediction model, by input of the measurement data received by the receiving unit. The prediction unit includes a determination unit that determines the quality of the drug substance, based on the predicted value. The quality prediction data includes a determination result obtained by determining the quality of the drug substance.

The estimation device according to the ninth item can present the prediction result for the quality of the drug substance in a more easily understandable manner.

(Tenth item) The estimation device according to any one of items 1 to 8, wherein the receiving unit further receives input of condition data indicating a culture condition of the cells. The prediction unit generates quality prediction data, by inputting measurement data and condition data received by the receiving unit, into a prediction model.

In the estimation device according to the tenth item, the prediction result in consideration of the relationship between the difference in culture condition and the quality is obtained, and a more correct prediction result is obtained.

(Eleventh item) A learning device according to an aspect includes: a receiving unit that receives training data; and a model generation unit that generates a prediction model by executing a learning process using the training data received by the receiving unit. The training data includes measurement data including a measurement result obtained by measuring at least one substance in a culturing vessel containing cells and a medium at a plurality of timings after seeding of the cells in the medium, and quality data obtained by analyzing a drug substance of a biopharmaceutical manufactured from the cells. The prediction model is a model for generating quality prediction data indicating the quality of a drug substance of a biopharmaceutical manufactured from cells contained in a culturing vessel during culture of the cells, based on a measurement result obtained by measuring at least one substance in the culturing vessel during culture of the cells.

In the stage during the culture, the learning device according to the eleventh item can generate the prediction model for predicting the quality of the drug substance obtained by progress of the culture. Accordingly, based on the predicted quality of the drug substance, a user can take various measures, such as reviewing the protocol in the stage of the culture step before the purification step for obtaining the drug substance, extending the culture time period, or stopping the culture. As a result, the biopharmaceutical manufacturing cost can be reduced.

(Twelfth item) In the learning device according to an aspect, training data further includes condition data indicating the culture condition of the cells, the condition data includes a plurality of parameter values that define the culture condition, the learning device further includes an optimization unit that optimizes a combination of a plurality of parameter values, and the optimization unit estimates an optimal combination of the plurality of parameter values, using, , as an input, the plurality of parameter values and the quality data obtained during culture of cells, based on the plurality of parameter values.

The learning device according to the twelfth item can estimate the combination of a plurality of parameter values that define the culture condition of cells in a stage during culture. Accordingly, the user can optimize the condition for improving the quality of the drug substance obtained by progress of the culture in the stage during the culture.

(Thirteenth item) In the learning device according to the eleventh item, the training data further includes condition data that indicates a condition for culturing cells contained in the culturing vessel. The training data received by the receiving unit includes first training data obtained when the cells are cultured under a first condition, and second training data obtained when the cells are cultured under a second condition that is different from the first condition. The model generation unit generates the prediction model by executing the learning process using the first training data and the second training data.

The learning device according to the thirteenth item can generate the prediction model in consideration of the relationship between the difference in culture condition and the temporal change in the substance in the culturing vessel, or the relationship between the difference in culture condition and the quality of the drug substance. Accordingly, the learning device can generate the prediction model for obtaining a more correct prediction result.

(Fourteenth item) An estimation method according to an aspect includes: putting cells and a medium in a culturing vessel, and culturing the cells; measuring at least one substance in the culturing vessel at least one timing after a predetermined time period has passed since the cells have been seeded in the medium; generating quality prediction data indicating a quality of a drug substance, by inputting the measurement data including a measurement result obtained in the measuring, into a prediction model; and outputting the quality prediction data. The prediction model is a model for predicting the quality of the drug substance of a biopharmaceutical manufactured by culturing the cells.

In the estimation method according to the fourteenth item, the quality of the drug substance obtained by progress of the culture in a stage during the culture can be predicted. Accordingly, based on the predicted quality of the drug substance, a user can take various measures, such as reviewing the protocol in the stage of the culture step before the purification step for obtaining the drug substance, extending the culture time period, or stopping the culture. As a result, the biopharmaceutical manufacturing cost can be reduced.

(Fifteenth item) A learning method according to an aspect includes: putting cells and a medium in a culturing vessel, and culturing the cells; measuring at least one substance in the culturing vessel at a plurality of timings after the cells are seeded in the medium; analyzing a quality of a drug substance of a biopharmaceutical manufactured from the cells; and generating a prediction model by executing a learning process using training data. The training data includes measurement data including a measurement result obtained by the step of measuring, and quality data obtained by the step of analyzing. The prediction model is a model for generating quality prediction data indicating the quality of a drug substance of a biopharmaceutical manufactured from cells contained in a culturing vessel during culture of the cells, based on a measurement result obtained by measuring at least one substance in the culturing vessel during culture of the cells.

In the stage during the culture, the learning method according to the fifteenth item can generate a prediction model for predicting the quality of the drug substance obtained by progress of the culture. Accordingly, based on the predicted quality of the drug substance, a user can take various measures, such as reviewing the protocol in the stage of the culture step before the purification step for obtaining the drug substance, extending the culture time period, or stopping the culture. As a result, the biopharmaceutical manufacturing cost can be reduced.

(Sixteenth item) An optimization device according to an aspect includes: a receiving unit that receives a plurality of parameter values that define a culture condition for seeding cells in a medium, and quality data obtained by analyzing a drug substance of a biopharmaceutical manufactured by culturing the cells; an estimation unit that receives the plurality of parameter values and the quality data received by the receiving unit, and estimates an optimal combination of the plurality of parameter values; and an output unit that outputs the combination of the plurality of parameter values estimated by the estimation unit.

The optimization device according to the sixteenth item can estimate the combination of a plurality of parameter values that define the culture condition of cells in a stage during culture. Accordingly, the user can optimize the condition for improving the quality of the drug substance obtained by progress of the culture in the stage during the culture. Note that the estimation unit may estimate the optimal combination of the plurality of parameter values by adopting, as input, a plurality of parameter values, and the quality data obtained by the step of analyzing, and executing the Bayesian optimization.

(Seventeenth item) An optimization method according to an aspect includes: putting cells and a medium in a culturing vessel, and culturing the cells under a culture condition defined by a plurality of parameter values; analyzing a quality of a drug substance of a biopharmaceutical manufactured by culturing the cells; and estimating an optimal combination of the plurality of parameter values using, as an input, the plurality of parameter values and the quality data obtained by the analyzing. The culturing the cells adopts the optimal combination of the plurality of parameter values estimated by the estimating, as a new culture condition, and cultures the cells under the new culture condition.

In the estimation method according to the seventeenth item, the condition for improving the quality of the drug substance obtained by progress of the culture in the stage during the culture can be optimized.

(Eighteenth item) A prediction system according to an aspect includes: the estimation device according to any one of the first to tenth items; the learning device according to any one of the eleventh to thirteenth items; and a measurement device that measures at least one substance in the culturing vessel.

Note that in the prediction system according to the eighteenth item, the estimation device and the measurement device may be implemented by a single information processing device. In this case, the receiving unit of the estimation device, and the receiving unit of the measurement device may be implemented as a single receiving unit. That is, the receiving unit receives the measurement data obtained by the measurement device measuring the substance.

(Nineteenth item) In the prediction system according to the eighteenth item, the measurement device includes a first measurement device that measures a first substance, and a second measurement device that measures a second substance.

Each of the embodiments disclosed this time are assumed to be implemented in a manner of combination in a range without technical contradiction. The embodiments disclosed this time are only examples in every respect, and should not be construed to be limitative. The scope of the present invention is not indicated by the description of the aforementioned embodiments but is indicated by the clams, and is intended to encompass all the changes within the meaning and scope equivalent to those of claims.

### REFERENCE SIGNS LIST

1 Cells, 2 Medium, 3 Drug substance, 10 Culturing vessel, 21, 31, 61 Processor, 22, 32, 62 Main memory, 23, 33, 63 Storage, 24, 34, 64 Communication I/F, 25, 35, 65 USB I/F, 26, 36, 66 Input unit, 27, 37, 67 Display unit, 28, 38, 68 Processor bus, 100 Measurement device, 200, 200a Estimation device, 210, 210a, 310 Receiving unit, 220, 220a Prediction unit, 222 Estimation process unit, 224 Determination unit, 230 Output unit, 300, 300a Learning device, 312 Unit of generating training data, 320, 320a Model generation unit, 330 Timing identification unit, 400 Server, 420, 420a Prediction model, 510 Measurement data, 512 Series data, 520 Quality data, 530, 530a, 530b Training data, 540 Quality prediction data, 542 Predicted value, 544 Determination result, 550 Condition data, 560 Estimation program, 580 Learning program, 600 Optimization device, 601 Receiving unit, 602 Estimation unit, 603 Output unit, 604 Storage unit, 620 Optimal parameter values, 630 Optimization program, 6000 Optimization unit, SYS1, SYS2 Prediction system, W10 Window.

## Claims

1. An estimation device, comprising:
a receiving unit that receives input of measurement data including a measurement result obtained by measuring at least one substance in a culturing vessel containing cells and a medium, at least one timing after a predetermined time period has passed since the cells have been seeded in the medium;
a prediction unit that generates quality prediction data indicating a quality of a drug substance, by inputting the measurement data received by the receiving unit, into a prediction model for predicting the quality of the drug substance of a biopharmaceutical manufactured by culturing the cells; and
an output unit that outputs the quality prediction data generated by the prediction unit.

2. The estimation device according to claim 1, wherein the measurement data includes a measurement result obtained by measuring the at least one substance at a plurality of timings after seeding of the cells in the medium.

3. The estimation device according to claim 1 or 2, wherein the measurement data includes a measurement result obtained by measuring the at least one substance at a start of culturing the cells.

4. The estimation device according to any one of claims 1 to 3,
wherein the measurement data includes a measurement result obtained by measuring the at least one substance in a logarithmic growth phase of the cells.

5. The estimation device according to any one of claims 1 to 4,
wherein the measurement data includes a measurement result obtained by measuring the at least one substance in a stationary phase of the cells.

6. The estimation device according to any one of claims 1 to 4,
wherein the measurement data includes a measurement result obtained by measuring the at least one substance in a death phase of the cells.

7. The estimation device according to any one of claims 1 to 6,
wherein the at least one substance is at least one of a nutrient ingested by the cell, a metabolite generated by metabolism of the cells, and the cells.

8. The estimation device according to any one of claims 1 to 7,
wherein a first predicted value in a case where the quality of the drug substance is evaluated from a first perspective, and a second predicted value in a case where the quality of the drug substance is evaluated from a second perspective are output from the prediction model, by input of the measurement data received by the receiving unit, and
the quality prediction data includes the first predicted value, and the second predicted value.

9. The estimation device according to any one of claims 1 to 7,
wherein a predicted value in a case where the quality of the drug substance is evaluated from a predetermined perspective is output from the prediction model, by input of the measurement data received by the receiving unit, and
the prediction unit includes a determination unit that determines the quality of the drug substance, based on the predicted value, and
the quality prediction data includes a determination result of determination of the quality of the drug substance.

10. The estimation device according to any one of claims 1 to 9,
wherein the receiving unit further receives input of condition data indicating a culture condition of the cells, and
the prediction unit generates the quality prediction data by inputting the measurement data and the condition data received by the receiving unit, into the prediction model.

11. A learning device, comprising:
a receiving unit that receives training data that includes measurement data including a measurement result obtained by measuring at least one substance in a culturing vessel containing cells and a medium at a plurality of timings after seeding of the cells in the medium, and quality data obtained by analyzing a drug substance of a biopharmaceutical manufactured from the cells; and
a model generation unit that generates a prediction model for generating quality prediction data indicating a quality of the drug substance of the biopharmaceutical manufactured from the cells contained in the culturing vessel during culture of the cells, by executing a learning process using the training data received by the receiving unit, based on a measurement result obtained by measuring the at least one substance in the culturing vessel during the culture of the cell.

12. The learning device according to claim 11,
wherein the training data further includes condition data indicating a culture condition of the cells,
the condition data includes a plurality of parameter values that define the culture condition,
the learning device further includes an optimization unit that optimizes a combination of the plurality of parameter values, and
the optimization unit estimates an optimal combination of the plurality of parameter values, using, as an input, the plurality of parameter values and the quality data obtained based on the plurality of parameter values when the cells are cultured.

13. The learning device according to claim 11,
wherein the training data further includes condition data indicating a culture condition of the cells,
the training data received by the receiving unit includes first training data obtained when the cells are cultured under a first culture condition, and second training data obtained when the cells are cultured under a second culture condition, and
the model generation unit generates the prediction model by executing the learning process using the first training data and the second training data.

14. An estimation method, comprising:
putting cells and a medium in a culturing vessel, and culturing the cells;
measuring at least one substance in the culturing vessel at least one timing after a predetermined time period has passed since the cells have been seeded in the medium; and
generating quality prediction data indicating a quality of a drug substance of a biopharmaceutical manufactured by culturing the cells, by inputting the measurement data including a measurement result obtained in the measuring, into a prediction model for predicting the quality of the drug substance; and
outputting the quality prediction data.

15. A learning method, comprising:
putting cells and a medium in a culturing vessel, and culturing the cells;
measuring at least one substance in the culturing vessel at a plurality of timings after the cells are seeded in the medium;
analyzing a quality of a drug substance of a biopharmaceutical manufactured by culturing the cells; and
generating a prediction model for generating quality prediction data indicating a quality of the drug substance of the biopharmaceutical manufactured from the cells contained in the culturing vessel during culture of the cells, by executing a learning process using training data that includes measurement data including a measurement result obtained by the measuring, and quality data obtained by the analyzing, based on a measurement result obtained by measuring the at least one substance in the culturing vessel during the culture.

16. An optimization device, comprising:
a receiving unit that receives a plurality of parameter values that define a culture condition for seeding cells in a medium, and quality data obtained by analyzing a drug substance of a biopharmaceutical manufactured by culturing the cells;
an estimation unit that receives the plurality of parameter values and the quality data received by the receiving unit, and estimates an optimal combination of the plurality of parameter values; and
an output unit that outputs the combination of the plurality of parameter values estimated by the estimation unit.

17. An optimization method, comprising:
putting cells and a medium in a culturing vessel, and culturing the cells under a culture condition defined by a plurality of parameter values;
analyzing a quality of a drug substance of a biopharmaceutical manufactured by culturing the cells; and
estimating an optimal combination of the plurality of parameter values using, as an input, the plurality of parameter values and the quality data obtained by the analyzing,
wherein the culturing the cells adopts the optimal combination of the plurality of parameter values estimated by the estimating, as a new culture condition, and cultures the cells under the new culture condition.
